# EUROPEAN PATENT SPECIFICATION

(11) **EP 0 651 760 B1**
(45) Date of publication and mention of the grant of the patent: **18.04.2001**
(21) Application number: 93922096.8
(22) Date of filing: 07.10.1993
(51) Int. Cl.: C07J 63/00, C07H 15/256, A61K 31/56

(54) **BIOACTIVE TRITERPENOIDS AND SECO-TRITERPENOIDS**
BIOAKTIVE TRITERPENOIDE UND SEC0-TRITERPENOIDE
TRITERPENOIDES ET SECO-TRITERPENOIDES BIOACTIFS

(30) Priority: 08.10.1992 PT 10093992
(43) Date of publication of application: 10.05.1995
(73) Proprietor: INSTITUTO NACIONAL DE ENGENHARIA E TECNOLOGIA INDUSTRIAL, 1699 Lisboa Codex (PT)
(72) Inventor: MOITEIRO, Cristina, Maria, Martins, P-2675 Odivelas (PT); ROSA, Maria Regina Tavares, Carcavelos, P-2775 Parede (PT); MARCELO CURTO, Maria, JoAo, P-2780 Oeiras (PT)
(74) Representative: HOFFMANN - EITLE
(86) International application number: PT9300006
(87) International publication number: WO9409026

(56) References cited:
- FR-A- 2 281 103
- INDIAN JOURNAL OF CHEMISTRY vol. 28B, May 1989, pages 376 - 380 A. PATRA ET AL 'Studies on Triterpenoids: Conversion of Friedelanones into Some Secofriedelanes'
- JOURNAL OF THE CHEMICAL SOCIETY. PERKIN TRANSACTIONS I no. 7, 1975, LETCHWORTH GB, pages 617 - 619 WAI-HAAN HUI ET AL 'Structure of Lithocarpic Lactone, a New Triterpenoid from Two Lithocarpus Species of Hong Kong'
- PHYTOCHEMISTRY vol. 24, no. 9, 1985, pages 1875 - 1889 W. J. BAAS 'Naturally Occurring Seco-Ring-A-Triterpenoids and Their Possible Biological Significance'
- INDIAN JOURNAL OF CHEMISTRY vol. 22B, August 1983, pages 741 - 745 B. TALAPATRA ET AL 'Terpenoids and Related Compounds: Part XXIII- Mechanism of Some Transformation Reactions of Friedelin & Its Derivatives'
- SYNTHESIS. no. 9, September 1979, STUTTGART DE, pages 736 - 738 I. FLEMING ET AL 'A Simple Synthesis of Carvone Using Silyl Enol Ethers'
- CHEMICAL ABSTRACTS, vol. 94, no. 21, 25 May 1981, Columbus, Ohio, US; abstract no. 168551, W. NES ET AL 'Effects of Tetracyclic and Pentacyclic Triterpenoids on Growth of Phytophthora cactorum' page 159; column 2; & J. NATURAL PRODUCTS vol. 44, no. 2, 1981, pages 215 - 220
- CHEMICAL ABSTRACTS, vol. 107, no. 15, 12 October 1987, Columbus, Ohio, US; abstract no. 129073, H. MILES 'Alkaloidal Insect Antifeedants from Virola calophylla Warb' page 259; column 2; & J. AGRIC. FOOD CHEM. vol. 35, no. 5, 1987, pages 794 - 797
- CHEMICAL ABSTRACTS, vol. 87, no. 19, 07 November 1977, Columbus, Ohio, US; abstract no. 147016, M. A. ABBASSY ET AL 'A New Antifeedant to Spodoptera littoralis Boisd. (Lepid., Noctuidae) from Acokanthera spectabilis Hook. (Apocynaceae).' page 155; column 2; & Z. ANGEW. ENTOMOL. vol. 83, no. 3 , 1977 pages 317 - 322
- JOURNAL OF THE CHEMICAL SOCIETY. PERKIN TRANSACTIONS I no. 12, December 1986, LETCHWORTH GB, pages 2117 - 2121 M. ANASTASIA ET AL 'Synthesis of (2R,3S,22S,23S)-2,3,22,23-Tetrahydroxy-B-ho mo-7-aza-5.alpha.-stigmastan-6-one, an Aza-analogue of Homobrassinolide'
- TETRAHEDRON LETTERS. vol. 22, no. 7, 1981, OXFORD GB, pages 607 - 610 J. P. MCCORMICK ET AL 'alpha-Hydroxylation of Ketones: Osmium Tetroxide / N-Methylmorpholine-N-Oxide Oxidation of Silyl Enol Ethers'
- BULL. CHEM. SOC. JPN vol. 60, 01 January 1987, pages 2117 - 2126
- TETRAHEDRON LETTERS vol. 30, 01 January 1989,, pages 5463 - 5466
- CHEM. REV. vol. 80, 01 January 1980,, pages 187 - 213
- H. HOUSE: "Modern Synthetic Reactions, 2nd Ed" , 01-01-1972, BENJAMIN/CUMMINGS PUBLISHING CO, MENLO PARK, CALIFORNIA, US
- 'Silylating Reagents', 01 January 1988, FLUKA CHEMIA A.G., CH
- R. T. MORRISON ET AL 'Lehrbuch der Organischen Chemie', 01 January 1984, VERLAG CHEMIE

## Description

### Background of the invention

This invention relates to processes for preparing friedelan triterpenoid derivatives which have application as bioactive agents in fungicidal, bactericidal, antitumour and antifeedant compositions, a precursor for preparing them, and a process for the production of said precursor from friedelin obtained from cork smoker wash solids.

Cork smoker wash solids are a by-product from the manufacture of cork particle boards, forming when cork granules are subject to compression and treatment with super-heated steam, and it accummulates in the piping as a black waxy material which forces operations to stop periodically for cleaning. Although cork smoker wash solids are a superior source of friedelin (V) and 3-hydroxyfriedel-3-en-2-one (VI) (R=H) [E. J. Corey and J. J. Ursprung, *J. Amer. Chem. Soc.,* 1956, 78, 5041; I. Ribas-Marques and J. Fernandez-Salgado, *Anal. Quím*., 1974, 70, 363] S. K. Talapatra, D. K. Pradhan and B. Talapatra, *Indian J. Chem.,* 1978, 16B, 361], up to the present time no extraction process has been exploited for industrial application and therefore this industrial waste has found no use, being usually destroyed by incineration.

The application of friedelin (V) has been described for the treatment of bladder cancer [H. C. Simon, German Patent 25 08 338, 19 Feb. 1976] and cachexia due to 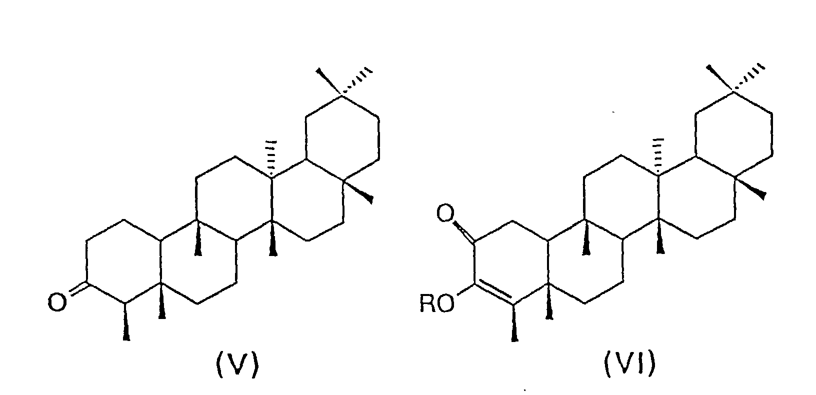 carcinoma, sarcoma and leukemia [H. C. Simon, US Patent 4,444,791, 24 Apr. 1984]. Its anticonvulsive, anti-inflamatory [A. K. Chaturvedi, S.S. Parmar, S. C. Bhatnagar, G. Mistra and S. K. Nigam, *Res. Commun. Chem. Pathol. Pharmacol*., 1974, 9, 11] and antidiuretic activities have been described [S. H. Rizvi, A. Shoeb, R. S. Kapil and S. P. Popli, *Experientia,* 1980, 36, 146]. Friedelin V has been reported to exhibit antifeedant activity for *Spodoptera littoralis* [M. A. Abbassy, A. El-Shazli and F. El-Gayar, Z. *Angew. Entomol.,* 1977, 83, 317] and *Anthonomus grandis* [D. H. Miles, A. M. Ly, S.
A. Randle, P. A. Hedin and M. L. Burks, *J. Agric. Food Chem.,* 1987, 35, 794], but these results were not confirmed by our own assay tests. Moreover, there are no references to the use of the new and known friedelin derivatives of the present invention as bioactive agents.

Accordingly, what is needed in the art is the high yield transformation of friedelin as isolated from cork smoker wash solids into new derivatives, or known derivatives from other sources unavailable up to the present in limited quantities, for application as effective bioactive agents. Quite surprinsigly, the present invention fulfills these needs.

### Summary of the invention

The present invention relates to triterpenoids of the general formula (I), 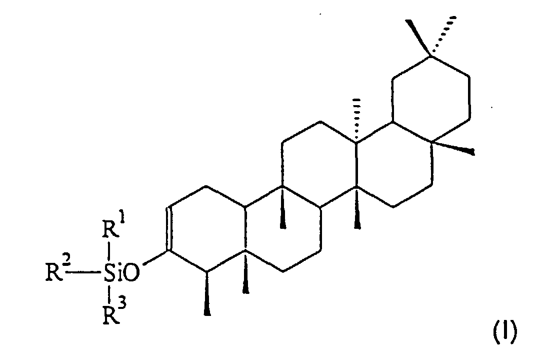 where R¹, R² and R³ represent each phenyl or alkyl, with R¹, R² and R³ identical or different.

Further, the present invention relates to process for the preparation of compound (I) as defined above wherein R¹, R² and R³ are methyl, which comprises the reaction of friedelin (V) with bis(trimethylsilyl)acetamide in the presence of sodium and hexamethylphosphoramide under kinetic control to produce regio-specifically the compound of formula (I) wherein R¹, R² and R³ are methyl.

Further, the present invention relates to process for the preparation of a compound of formula (III), as shown below, which comprises reaction of compound (I) as defined above with an oxidizing agent to produce the intermediate of formula (VII), as shown below. The compound of formula (VII) may be further oxidized with a peracid to produce quantitatively the compound of formula (III), as shown below, which is optionally reacted with alcohols or bases according to well known methods to yield the corresponding esters or salts. 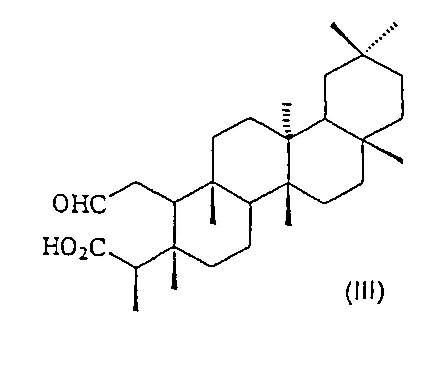 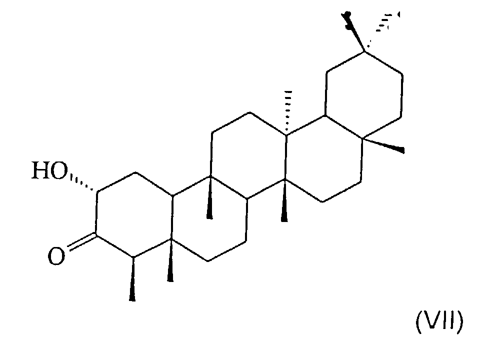 Further, the present invention relates to process wherein the compound of general formula (I) as defined above is subjected to ozonolysis followed by reductive treatment to produce the compound of formula (IX) as shown below. 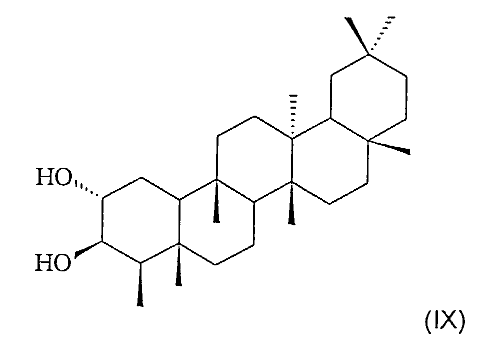 The present invention also involves process where a compound of general formula (I) as defined above is subjected to oxidation to produce friedel-1-en-3-one (XI) as shown below.

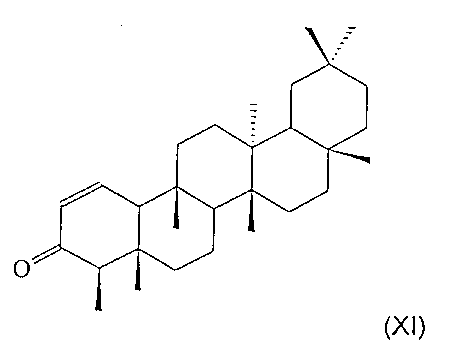

### Figure

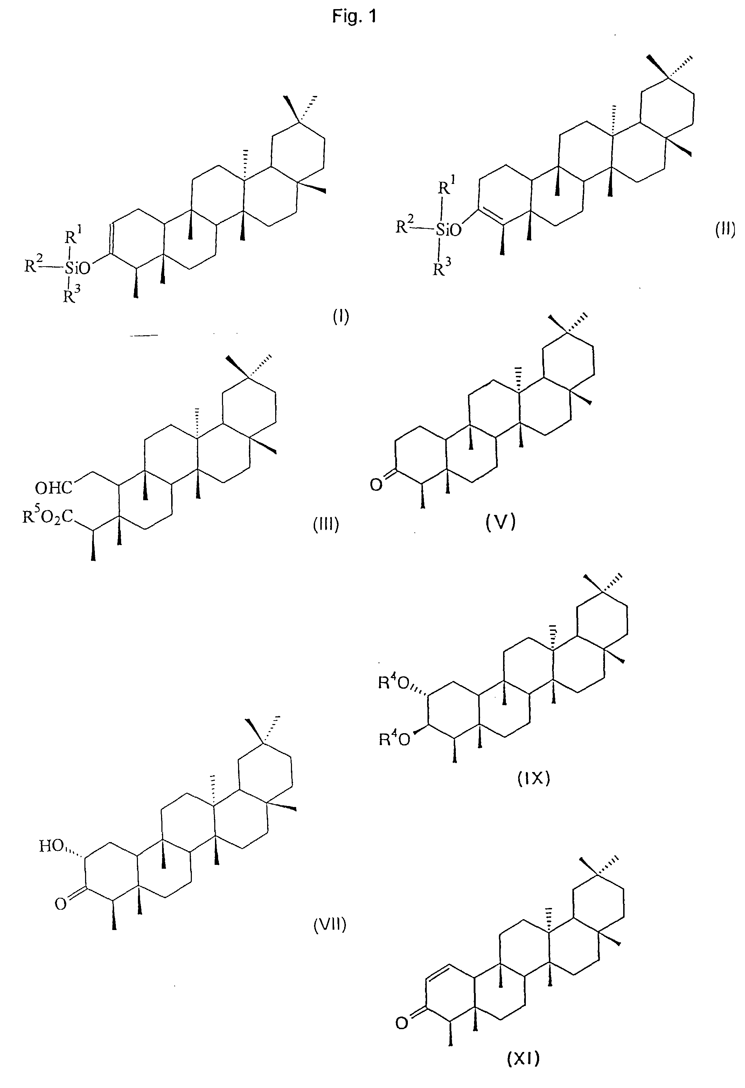

Figure 1 shows the constitution formulae of compounds (I), (II), (III), (V), (VII), (IX) and (XI).

### Description of the Specific Embodiments

In the above compound (I) of the invention R¹, R² and R³ represent each phenyl or alkyl. SiR¹R²R³ represents for instance trialkylsilyl, triphenylsilyl, diphenylalkylsilyl, alkyldiphenylsilyl, diakylphenylsilyl. R²O₂C represents a carboxyl group, salt or ester. In the reference compound (II), R¹, R² and R³ have the same meaning. Potential meanings of R⁴ and R⁵ are explained in Example 4 and 3, respectively.

One process of the present invention provides the compounds of general formula (I), which can be produced from friedelin (V) by regio-specific silylation in no less than 85% yield, by reaction in basic medium with sodium in hexamethylphosphoramide as a bulky strong base and bis(trimethylsilyl)acetamide as silylating agent; time and temperature control determines the formation of the kinetic product, i.e. the new silyl enol ether (I)
On the other hand, it is possible to produce the silyl enol ether (II) with the same base and the same silylating agent under thermodynamic control.

The present process for the production (I) is a significant improvement over earlier methods for a number of reasons which include: (1) although the type of process has been previously described, it was only applied to straight chain ketones up to 7 carbon atoms and for camphor [J. Dedeier, P. Gerval and E. Frainnet, J. Organometal, *Chem.,* 1980, 185, 183]. (2) In spite of the fact that silylation of steroidal ketones with trimethylsilyl chloride in the presence of lithium isopropylamide in good yields has been described [see, for example, I. Fleming and I.Paterson, *Synthesis,* 1979, 736; M. Anastasia, P. Allevi, P. Ciuffreda, A. Fiecchi and A. Scale, *J. Chem. Soc. Perkin Trans. I*, 1986, 2117], friedelin V reacts with difficulty under such conditions.

According to one further process of the present invention, to achieve α-hydroxylation of friedelin (V), the enol silyl ether (I) is used as intermediate to yield, 2α-hydroxyfriedelan-3-one (VII, cerine) by means of an oxidising agent 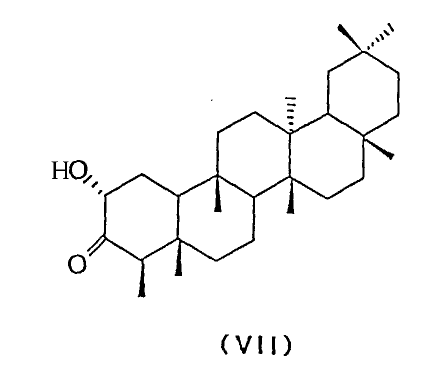 (in yields higher than 60%). This reaction may be based on the formation of α-ketols using catalytic amounts of osmium tetroxide in the presence of a stoicheometric oxidant [see, for example, J. P. McCormick, W. Tomasik and M. W. Johnson, *Tetrahedron Lett.,* 1981, 22, 607].

The process for the production of cerine (VII) by α-hydroxylation of friedelin (V) (via the intermediate (I)) is a significant improvement over an earlier method as described by G. Gutta and S. N. Bose, *Indian J. Chem.,* 1989, 28B, 975, where cerine (VII) was produced in ca. 20% by oxidation of friedelin (V) with lead tetraacetate followed by hydrolysis. Moreover, it is worth noting that cerine (VII) does not occur in cork smoker wash solids and is found only as traces in cork powder [see, for example, M. L. Mata, *Identificação dos Constituintes Químicos do Pó da Cortiça e sua Valorização por Hemissíntese,* M.Sc. Dissertation, Mestrado em Química Orgânica Tecnológica, Universidade Nova de Lisboa, Lisboa, 1991].

According to one further embodiment of the present invention the seco-acid of formula (III) (R⁵=H) is prepared 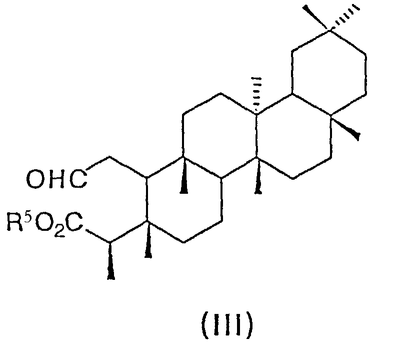 in quantitative yield from compound (VII) which is made to react with periodic acid [see, for example, M. Anastasia, P. Allevi, P. Ciuffreda, A. Fiecchi and A. Scala, *J. Chem. Soc. Perkin Trans. I*, 1986, 2117]. Compound (III) can, by reaction with alcohols or bases, according to methods well known to those skilled in the art, yield the corresponding seco-esters or salts. Although the conversion of friedelanones into seco-friedelanes has been described by A. Patra and S. K. Chaudhuri, *Indian J. Chem*., 1989, 28 B, 376, in the case of friedelin (V) and due to its low reactivity, 3,4-seco-friedelane-3,4-lactone was formed in yields not greater than 3% and there are no other reports of synthetic pathways leading to higher yields of seco-acids.

According to one further process the present invention, the diol of formula (IX) (R⁴=H) 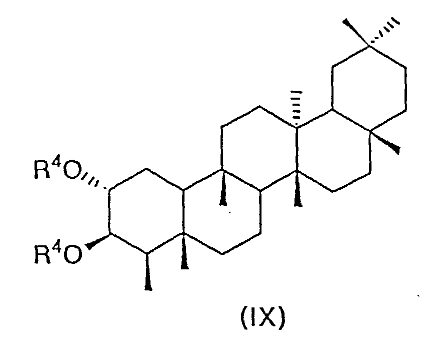 is prepared in high yields from the silyl enol ether (l), by ozonolysis [see, for example, R. D. Clark and C. H. Heathcock, *J. Org. Chem.,* 1976, 41, 1396] followed by reductive treatment according to known methods [see, for example, L. Aringer and P. Eneroth, *Steroids*, 1972, 18, 381], and the acyl or glycosyl derivatives thereof may be produced in excellent yields from the diol IX (R⁴=H) by acylation or glycosylation by methods well established.

Also according to another process of the present invention, the known compound of formula (XI) friedel-1-en-3-one, 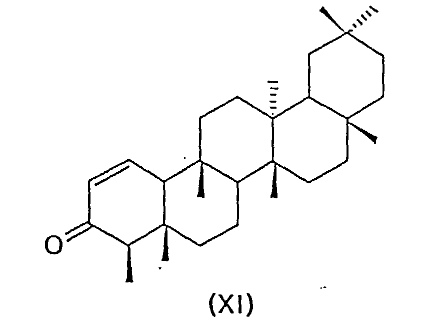 can be produced in yields higher than 75% by oxidation of the silyl enol ether of general formula (I) preferably with 2,3-dichloro-5,6-dicyano-1,4-benzoquinone (DDQ) [see, for example, I. leming and I. Paterson, *Synthesis,* 1979, 736].

The present process for the production of (XI) illustrates the importance of the silyl enol ether (I) as intermediate for synthesis and for the selective introduction of α,β-unsaturation in a saturated ketone and is a significant improvement over earlier methods, as described by V. Anjaneyulu and G. S. Rao, *Indian J. Chem.,* 1984, 23B, 663, by oxidation of friedelin (V) with hydrogen peroxide and selenium dioxide to produce XI in only 2.5% yield , and by B. Talapatra, B. Lahiri, A. Basak, D. K. Pradhan and S. K. Talapatra, *Indian J. Chem.,* 1983, 22B, 741, where XI is obtained in 20% yield starting from 2α-bromofriedel-3-one by reaction with lithium bromide and lithium carbonate.

The fungicidal and bactericidal activity of friedelin V, 3-hydroxyfriedel-3-en-2-one VI (R=H), of compounds (I), (II), (III), (VII), (IX) and (XI) and the acyl and glycosyl derivatives of (VII), (IX) and (XI) was demonstrated with bioautographic assay tests with *Cladosporium cucumerinum* and serology plate assay test with filamentous fungi, yeasts and bacteria, including *Streptococcus* sp. [for this method, see M. L. Quinta, S. Feio, B. Gigante and M. J. Marcelo Curto, *Abstracts II Jornadas Ibéricas de Plantas Medicinais, Aromáticas e Óleos Essenciais,* 1991], showing that in particular the ester of the seco-acids III has higher activity, possessing the structural requirements for the presence of biological activity, namely a rigid polycyclic system with a carboxyl group in the vicinity of an oxygenated function [see, for example, J. Fried, G. W. Krakower, D. Rosenthal and H. Basch, *J. Med. Chem*., 1965, 8, 279; I. Valterová, J. Klinot and A. Vystrcil, *Collect. Czech. Chem. Commun.,* 1983, 48, 649].

The antifeedant assay tests were carried out by methods well known to those skilled in the art [see, for example, D. H. Miles, A. M. Ly, S. A. Randle, P. A. Hedin and M. L. Burke, *J. Agric. Food Chem.,* 1987, 35, 794; F. N. Lugemwa, F.-Y. Huang, M. D. Bentley, M. J. Mendel and A. R. Alford, *J. Agric. Food Chem.,* 1990, 38, 493] with *Leptinitarsa decemlineata*, and other insect species of economic significance, such as *Epilachna varivestis, Helicoverpa zea* and *Spodoptera frugiperda.* In particular, the antifeedant activity of the friedelan derivatives has advantages over conventional agents used in pest control because they are readily biodegradable and its action is specific, an important requirement for their use in integrated pest control programs to protect agricultural crops of economic importance.

Compounds of formulae (III), (VII), (IX), and (XI), and the acyl and glycosyl derivatives of (VII), (IX) and (XI) can be used as bioactive agents or formulated in fungicidal, bactericidal, antitumour or antifeedant compositions.

The processes of the present invention are illustrated in the following Examples, starting from cork smoker wash solids since it is the most convenient source of friedelin (V), and are offered by way of illustration, not by way of limitation.

### EXAMPLES

### REFERENCE EXAMPLE 1

This reference example illustrates the isolation of friedelin (V) from cork smoker wash solids.

Powdered cork smoker wash solids (1.2 kg) were extracted with petroleum ether 40-60°C (3 L) for 2 h in a Soxhlet. The extract was concentrated at reduced pressure and the residue (230 g, 22.9%, w/w), after removal of waxes (4.3%) with petroleum ether, was fracctionated by column chromatography on silica with petrol ether:dichloromethane to yield friedelin V (8% w/w), m.p. 260-4°C, υₘₐₓ(KBr) 1710 (C=O) cm⁻¹, ¹H-NMR δ 1,69 (1H, *m*, Heq-1), 1,95 (2H, *m*, Hax-1), 2,24 (1H, *q*, Hax-4), 2,31 (1H, *sex,* Hax-2), 2,39 (1H, *ddd,* Heq-2) ppm; ¹³C-NMR δ 22,2 (C-1), 41,5 (C-2), 213,2 (C-3), 58,2 (C-4), 42,1 (C-5), 41,2 (C-6) ppm; m/z 426 [M]⁺ and 3-hydroxyfriedel-3-en-2-one VI (R=H) (5.4% w/w), m.p. 265-9°C, λₘₐₓ(EtOH) 275 nm; υₘₐₓ (KBr) 3380 (OH), 1660 (conjugated C=O), 1630 (C=C) cm⁻¹; ¹H-NMR δ (CDCl₃) 2,41 (1H, *dd*, Hax-1), 2,53 ( 1H, *dd,* Heq-1), 5,96 (1H, *s*, OH, exchanges with D₂O) ppm; ¹³C-NMR δ (CDCl₃)32,1 (C-1), 194,8 (C-2), 142,2 (C-3), 140,5 (C-4), 39,6 (C-5), 38,1 (C-6) ppm; m/z 440 [M]⁺.

### EXAMPLE 1

This example illustrates the preparation of 3-trimethylsiloxy-friedel-2-ene (I, R¹, R², and R³=Me) and, as reference compound, 3-timethylsiloxy-friedel-3-ene (II, R¹ R² and R³ = Me)

To a mixture of friedelin(V) (0,53 g) and bis(trimethylsilyl)acetamide (1,0 ml) under nitrogen and with stirring, was added hexamethylphosphoramide (1,0 ml) and sodium. After ca. 0.5 h, the mixture was diluted with n-pentane and iced water, the organic phase extracted, dried and the solvent removed at reduced pressure to yield 3-trimethylsiloxyfriedel-2-ene (I, R¹ R² and R³=Me) (0.54 g, 87%), m.p. 188-190°C. The structure of this new compound was established by IR, ¹H and ¹³C-NMR and MS: υₘₐₓ (CHCl₃) 1670 (C=C), 1440, 1370, 1090, 1050 (SiOC), 900, 840 (SiC) cm⁻¹; ¹H-NMR δ (CDCl₃) 0,17 (9H, *s*, OSi(CH₃)₃), 1,89 (3H, *m*, 2H-1, H-4), 4,78 (1H, *t, J* =1,2, CH=COSi(Me)₃) ppm; ¹³C-NMR δ (CDCl₃) 0,30 ((CH₃)₃Si), 20,8 (C-1), 102,5 (C-2), 151,3 (C-3), 53,0 (C-4), 41,8 (C-6) ppm; m/z 498 [M]⁺, 439 [M-(Si(Me)₂+H)]⁺, 75 [(Me)₂Si=OH]⁺, 73 [(Me)₃Si]⁺.

The more substituted silyl enol ether, 3-trimethylsiloxy-friedel-3-ene (II, R¹ R² and R³=Me) was obtained in a similar way, at 30-50°C and with prolonged reaction times. The structure of this new compound was established by IR, ¹H and ¹³C-NMR and MS: υₘₐₓ (CHCl₃) 2920, 1660, 1595, 890 cm⁻¹; ¹H-NMR δ (CDCl₃) 0,17 (9H, *s*, OSi(CH₃)₃), 0,16 (9H, *s*, OSi(CH₃)₃), 4,78 (1H, *t*, CH=COSiMe₃) ppm; m/z 498 [M]⁺, 439 [M-(Si(Me)₂+H)]⁺, 75 [(Me)₂Si=OH]⁺, 73 [(Me)₃Si]⁺.

### EXAMPLE 2

This example illustrates the preparation of 2α -hydroxyfriedelan-3-one (cerine, VII)

3-Trimethylsiloxy-friedel-2-ene (I, R¹ R² and R³ = Me) (0.4 g, 0.8 mmol) dissolved in acetone (3 ml) at -5°C was added to a mixture of osmium tetroxide (40,8 mg) in t- butyl alcohol (0.3 ml) and N-methylmorpholine N-oxide (0.114 g) in water and acetone (5 ml, 1:3). After stirring at 0°C for 3 h, the reaction mixture was kept at room temperature for 8 h. Sodium bisulphite (0.14 g) and Florisil (0.54 g) were added and the suspension stirred for 30 min. After filtration and removal of acetone at reduced pressure, pH was brought to ca. 2, sodium chloride added and the mixture extracted with ethyl acetate, the organic layer dried and concentrated at reduced pressure to give a white solid (0.33 g) which was purified by chromatography on silica to afford 20-hydroxyfriedelan-3-one (cerine, VII), white powder (0.21 g, 59%), m.p. 230°C, υₘₐₓ(CHCl₃) 3608 (OH), 1715 (C=O), 1457, 1391, 1031 (OH) cm⁻¹; ¹H-NMR δ (CDCl₃) 1,97 (2H, *m*, H-1), 2,18(1H, *d*, OH-2), 2,82 (1H, *q*, H-4), 4,08 (1H, *q*, H-2) ppm; ¹³C r.m.n. δ (CDCl₃) 32,4 (C-1), 76,9 (C-2), 212,0 (C-3), 52,6 (C-4), 42,7 (C-5) ppm; m/z 442 [M]⁺.

### EXAMPLE 3

This example illustrates the preparation of 2,3-seco-friedelan-2-al-3-oic acid (III) (R⁵=H) and methyl 2,3-seco-friedelan-2-al-3-oate (III, R⁵=CH₃). (R⁵ is shown in Fig. 1).

2α-Hydroxyfriedelan-3-one (cerine, VII), (0.7 g) was dissolved in diethyl ether (150 ml) and periodic acid (0.45 g) was added at 0°C, keeping the mixture with stirring for 1 h and then at room temperature for 3 h. Filtration and concentration at reduced pressure gave 2,3-seco-friedelan-2-al-3-oic acid (III, R⁵=H) (0.73 g, 100%). The structure of this new compound was established by IR, ¹H and ¹³C-NMR and MS of the corresponding methyl ester, methyl 2,3-seco-friedelan-2-al-3-oate (III, R⁵=CH₃), m.p. 156-61°C, υₘₐₓ(CHCl₃) 2735 (CHO), 1732 (C=O) cm⁻¹; 1H-NMR δ (CDCl₃) 2,29 (1H, *ddd,* Heq-1), 2,30 (1H, *q*, H-4), 2,45 (1H, *ddd,* Hax-1), 3,62 (3H, *s*, CO₂Me), 9,76 (1H, *s*, CHO) ppm; ¹³C-NMR δ (CDCl₃) 29,7 (C-1), 201,6 (C-2), 175,9 (C-3), 41,3 (C-4), 39,8 (C-5), 51,0 (C-31, ester) ppm; m/z 472 [M]⁺, 413 [M-OCOMe]⁺.

### EXAMPLE 4

This example illustrates the preparation of friedelane-2α,3β-diol (IX, R⁴= H) and 2α,3β-diacetoxyfriedelane (IX, R⁴=Ac), (R⁴ is shown in Fig. 1).

Ozone (30 ml/min) was bubbled for 1 h in a solution of 3-trimethylsiloxy-friedel-2-ene (I, R¹ R² and R³ =Me) (0.36 g) in dichloromethane and methanol (100 ml, 4:1) at -70°C. Sodium borohydride was added (53 mg) and stirring at room temperature kept for 12 h. After destruction of excess hydride with 10% hydrochloric acid, the mixture was extracted with dichloromethane, dried and concentrated at reduced pressure to give friedelane-2α,3β-diol (65%) (IX, R⁴=H), m.p. 270-3°C; υₘₐₓ (KBr) 3440 (OH) cm⁻¹; ¹H-NMR δ (CDCl₃) 1,58 (2H, *m*, Heq-1 and H-4), 1,83 (1H, *dt,* Hax-1), 3,54 (1H, *t,* Hax-3), 3,99 (1H, *q*, Heq-2); m/z 444 [M]⁺, 429 [M-Me]⁺, 410 [M-2xOH]⁺.

Acetylation of friedelane-2α,3β-diol (IX, R⁴=H) with excess acetic anhydride and pyridine gave quantitatively 2α,3β-diacetoxyfriedelane (IX, R⁴=Ac), m.p. 225-30°C; ¹H-NMR δ (CDCl₃) 2,06 and 2,08 (6H, s, 2x CH₃C=O), 4,79 (1H, *t*, H-3), 4,70 (1H, *t*, H-2) ppm; m/z 528 [M]⁺, 513 [M-Me]⁺, 468 [M-MeCOOH]⁺, 453 [M-(MeCOOH+Me)]⁺.

### EXAMPLE 5

This example illustrates the preparation of friedel-1-en-3-one (XI).

To a solution of 3-trimethylsiloxy-friedel-2-ene (I, R¹ R² and R³=Me) (0.17 g) in benzene (10 ml) with stirring was added dropwise a solution of DDQ (90 mg) in benzene (30 ml). After stirring at room temperature for 24 h, a saturated solution of sodium bicarbonate (30 ml) was added and the mixture extracted with diethyl ether, the organic layer separated, dried and concentrated at reduced pressure to yield a residue which was purified by chromatography to afford friedel-1-en-3-one XI (0.11 g, 75.9%), white needles m.p. 205-210°C ; λₘₐₓ (EtOH) 232,4 nm; υₘₐₓ(KBr) 1660 (C=O conjugado), 905 (CH=CH) cm⁻¹; ¹H-NMR δ (CDCl₃) 2,23 (1H, *q*, H-4), 2,24 (1H, *dd,* H-10), 6,05 (1H, *dd,* H-1), 6,94 (1H, *dd,* H-2) ppm; ¹³C-NMR δ (CDCl₃) 148,6 (C-1), 130,3 (C-2), 201,4 (C-3), 57,9 (C-4), 43,8 (C-5), 40,1 (C-6) ppm; m/z 424 [M]⁺.

## Claims

1. The triterpenoids of the general formula (I), 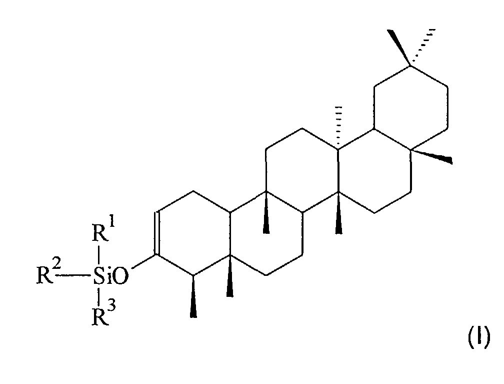 where R¹, R² and R³ represent each phenyl or alkyl, with R¹, R² and R³ identical or different.

2. A process for the preparation of compound (I) as defined in claim 1 wherein R¹, R² and R³ are methyl, which comprises the reaction of friedelin (V) with bis(trimethylsilyl)acetamide in the presence of sodium and hexamethylphosphoramide under kinetic control to produce regio-specifically the compound of formula (I) wherein R¹, R² and R³ are methyl.

3. A process for the preparation of a compound of formula (VII) which comprises reaction of Compound (I) as defined in claim 1 with an oxidizing agent to produce a compound of the following formula (VII).

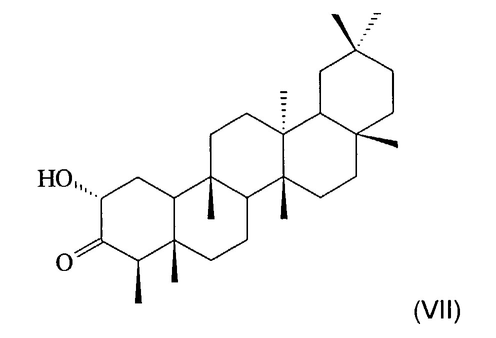

4. A process for the preparation of a compound of formula (III), which comprises reaction of compound (I) as defined in claim 1 with an oxidising agent to produce the intermediate of formula (VII) as given in claim 3, which is further oxidised with a peracid to produce quantitatively the compound of formula (III), which is optionally reacted with alcohols or bases according to well known methods to yield the corresponding esters or salts.

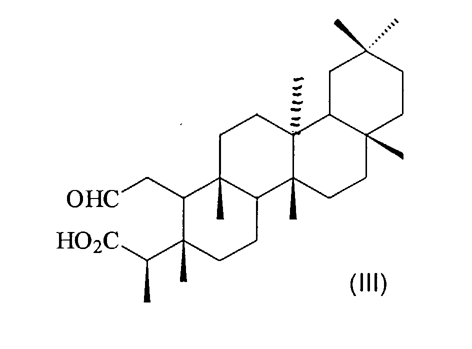

5. The process according to Claim 4, in which the preferred oxidising agent to produce the intermediate of formula (VII) is a stoichiometric oxidant in the presence of catalytic amounts of osmium tetroxide.

6. The process according to Claim 5, in which the preferred stoichiometric oxidant is N-methylmorpholine N-oxide.

7. The process according to Claim 4, in which the preferred peracid oxidising agent to oxidise intermediate (VII) to compound (III) is periodic acid.

8. A process wherein the compound of general formula (I) as defined in claim 1 is subjected to ozonolysis followed by reductive treatment to produce the compound of formula (IX).

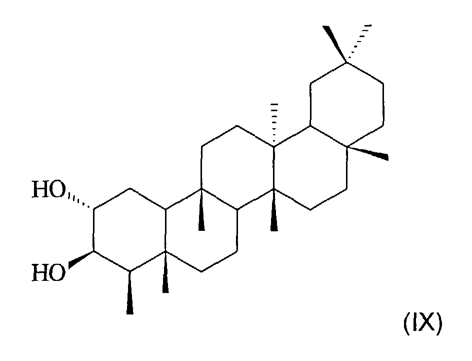

9. The process according to Claim 8, in which the preferred reducing agent is a metal hydride.

10. The process according to Claims 8 and 9, in which the compound of formula (IX) is acylated or glycosylated.

11. A process where a compound of general formula (I) as defined in claim 1 is subjected to oxidation to produce friedel-1-en-3-one (XI).

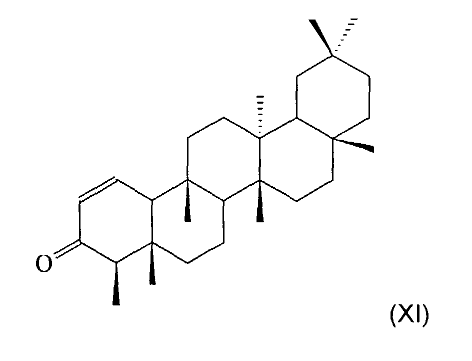

12. The process according to Claim 11, in which the preferred oxidising agent is DDQ.

## Patentansprüche

1. Triterpenoide der allgemeinen Formel (I) 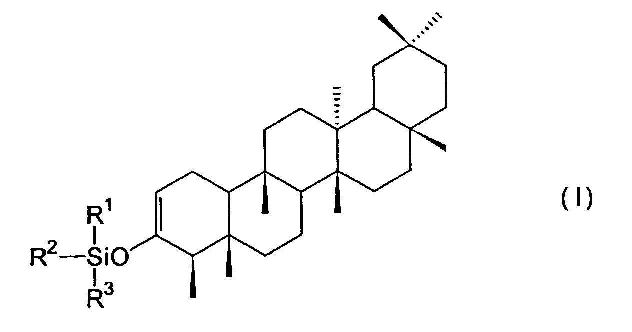 worin R¹, R² und R³ jeweils Phenyl oder Alkyl bedeuten, wobei R¹, R² und R³ gleich oder verschieden sind.

2. Verfahren zur Herstellung der Verbindung (I), wie sie in Anspruch 1 definiert ist, worin R¹, R² und R³ Methyl sind, welches die Reaktion von Friedelin (V) mit Bis(trimethylsilyl)acetamid in Gegenwart von Natrium und Hexamethylphosphoramid unter kinetischer Kontrolle umfasst, um regiospezifisch die Verbindung der Formel (I) zu bilden, worin R¹, R² und R³ Methyl sind.

3. Verfahren zur Herstellung einer Verbindung der Formel (VII), welches die Reaktion der Verbindung (I), wie sie in Anspruch 1 definiert ist, mit einem Oxidationsmittel umfasst, um eine Verbindung der folgenden Formel (VII) herzustellen:

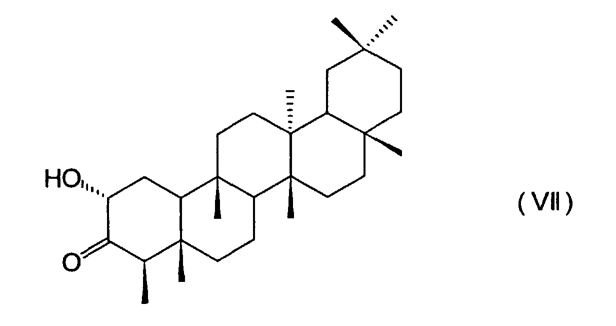

4. Verfahren zur Herstellung einer Verbindung der Formel (III), welches die Reaktion der Verbindung (I), wie sie in Anspruch 1 definiert ist, mit einem Oxidationsmittel umfasst, um die Zwischenstufe der Formel (VII), wie sie in Anspruch 3 angegeben ist, herzustellen, die mit einer Persäure weiteroxidiert wird, um quantitativ die Verbindung der Formel (III) herzustellen, die gegebenenfalls mit Alkoholen oder Basen nach wohlbekannten Verfahren umgesetzt wird, um die entsprechenden Ester oder Salze zu ergeben:

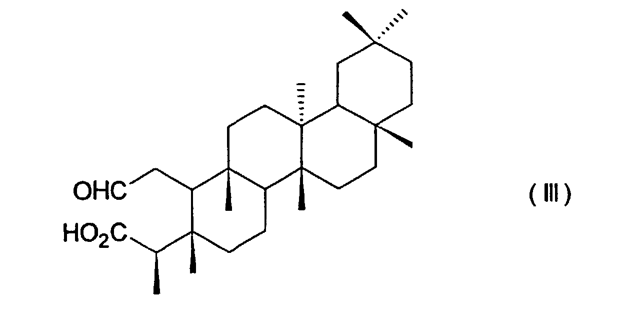

5. Verfahren gemäss Anspruch 4, in welchem das bevorzugte Oxidationsmittel zur Herstellung der Zwischenstufe der Formel (VII) ein stöchiometrisches Oxidationsmittel in Gegenwart von katalytischen Mengen von Osmiumtetroxid ist.

6. Verfahren gemäss Anspruch 5, in welchem das bevorzugte stöchiometrische Oxidationsmittel N-Methylmorpholin-N-oxid ist.

7. Verfahren gemäss Anspruch 4, worin das bevorzugte Persäure-Oxidationsmittel zur Oxidation der Zwischenstufe (VII) zur Verbindung (III) Periodsäure ist.

8. Verfahren, worin die Verbindung der allgemeinen Formel (I), wie sie in Anspruch 1 definiert ist, einer Ozonolyse, gefolgt von einer reduktiven Behandlung unterzogen wird, um die Verbindung der Formel (IX) herzustellen:

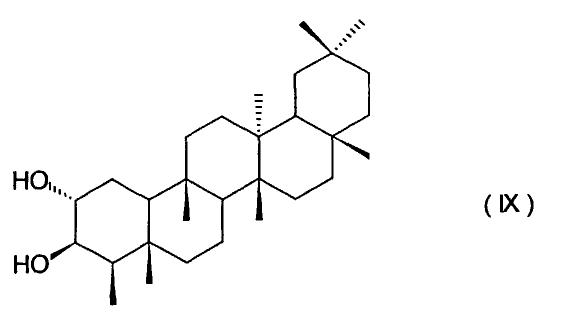

9. Verfahren gemäss Anspruch 8, worin das bevorzugte Reduktionsmittel ein Metallhydrid ist.

10. Verfahren gemäss Anspruch 8 und 9, worin die Verbindung der Formel (IX) acyliert oder glykosyliert wird.

11. Verfahren, worin eine Verbindung der allgemeinen Formel (I), wie sie in Anspruch 1 definiert ist, einer Oxidation unterzogen wird, um Friedel-1-en-3-on (XI) herzustellen:

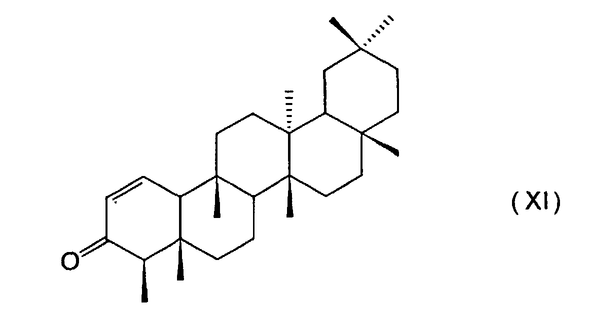

12. Verfahren gemäss Anspruch 11, worin das bevorzugte Oxidationsmittel DDQ ist.

## Revendications

1. Triterpénoïdes de formule générale (I), 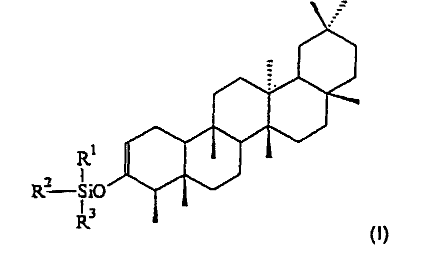 où R¹, R² et R³ représentent chacun un groupement phényle ou alcoyle, R¹, R² et R³ étant identiques ou différents.

2. Procédé de préparation du composé (I), comme défini dans la revendication 1, dans lequel R¹, R² et R³ représentent un groupement méthyle, qui comprend la réaction de la friedeline (V) avec le bis (triméthylsilyl) acétamide en présence de sodium et d'hexaméthylphosphoramide sous contrôle cinétique en vue de fabriquer régiospécifiquement le composé de formule (I) dans lequel R¹, R² et R³ représentent un groupement méthyle.

3. Procédé de préparation d'un composé de formule (VII) qui comprend la réaction du composé (I), comme défini dans la revendication 1, avec un agent oxydant en vue de fabriquer un composé de formule (VII) suivante.

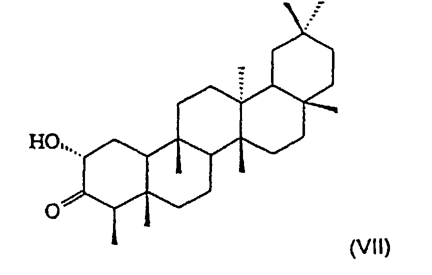

4. Procédé de préparation d'un composé de formule (III), qui comprend la réaction du composé (I), comme défini dans la revendication 1, avec un agent oxydant en vue de fabriquer l'intermédiaire de formule (VII) comme fourni dans la revendication 3, lequel est davantage oxydé avec un peracide en vue de fabriquer quantitativement le composé de formule (III) qui est optionnellement mis à réagir avec des alcools ou des bases conformément à des procédés bien connus en vue de produire les esters ou les sels correspondants.

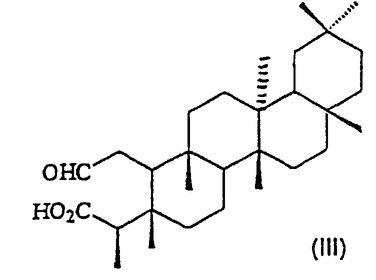

5. Procédé selon la revendication 4, dans lequel l'agent oxydant préféré pour fabriquer l'intermédiaire de formule (VII) est un oxydant stoechiométrique en présence de quantités catalytiques de tétroxyde d'osmium.

6. Procédé selon la revendication 5, dans lequel l'oxydant stoechiométrique préféré est le N-oxyde de N-méthylmorpholine.

7. Procédé selon la revendication 4, dans lequel l'agent oxydant de peracide préféré pour oxyder l'intermédiaire (VII) en composé (III) est l'acide periodique.

8. Procédé dans lequel le composé de formule générale (I), comme défini dans la revendication 1, est soumis à une ozonolyse suivie d'un traitement réducteur en vue de fabriquer le composé de formule (IX).

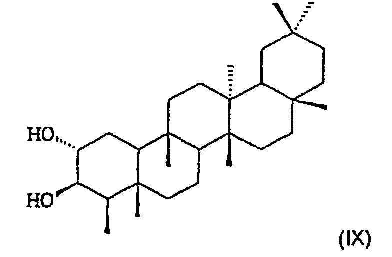

9. Procédé selon la revendication 8, dans lequel l'agent réducteur préféré est un hydrure métallique.

10. Procédé selon les revendications 8 et 9, dans lequel le composé de formule (IX) est acylé ou glycosylé.

11. Procédé où un composé de formule générale (I), comme défini dans la revendication 1, est soumis à une oxydation en vue de fabriquer la friedel-1-ène-3-one (XI).

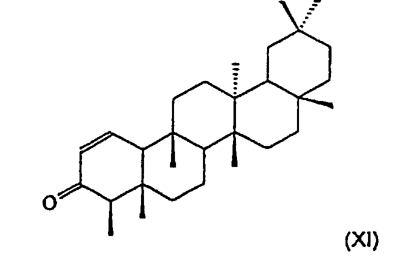

12. Procédé selon la revendication 11, dans lequel l'agent oxydant préféré est la DDQ.
